# EUROPEAN PATENT SPECIFICATION

(11) **EP 4 308 058 B1**
(45) Date of publication and mention of the grant of the patent: **04.12.2024**
(21) Application number: 21714115.9
(22) Date of filing: 18.03.2021
(51) Int. Cl.: A61H 9/00

(54) **DEVICE FOR CARRYING OUT A RELAXATION SHIRODHARA TREATMENT**
GERÄT ZUR DURCHFÜHRUNG EINER ENTSPANNENDEN SHIRODHARA-BEHANDLUNG
DISPOSITIF POUR LA RÉALISATION D'UN TRAITEMENT DE RELAXATION SHIRODHARA

(43) Date of publication of application: 24.01.2024
(73) Proprietor: Trischler, Heinrich, 8063 Eggersdorf bei Graz (AT)
(72) Inventor: Trischler, Heinrich, 8063 Eggersdorf bei Graz (AT)
(74) Representative: KLIMENT & HENHAPEL
(86) International application number: PCT/EP2021/057007
(87) International publication number: WO 2022/194379

(56) References cited:
- WO-A1-2019/179822
- WO-A2-2015/056281
- JP-A- H11 221 261
- US-A- 4 834 121
- US-B1- 6 402 776

## Description

### FIELD OF THE INVENTION

The invention relates to a device for carrying out a relaxation treatment on the forehead of a person to be treated by producing a continuous liquid stream, the device comprising
- an outlet nozzle for forming the liquid stream;
- a liquid reservoir which is designed to receive the liquid stream at least partially;
- a sliding unit for reciprocating movement of the outlet nozzle in a lateral direction;
- at least one circulation pump to convey liquid from the liquid reservoir to the outlet nozzle;
- a pipe system connecting the outlet nozzle and/or the liquid reservoir with the at least one circulation pump (5);
- a housing.

### PRIOR ART

Classic Shirodhara treatment is carried out by continuously pouring a warm fluid as oil, tee or buttermilk through a wick over the forehead of a person who is lying on the back. The cotton wick passes through traditionally a copper pot that is hanging on a chain above the head of the person and is moved manually by a practitioner in order for the liquid stream to move laterally over the forehead during treatment. The copper pot contains a hole in the middle of the bottom from where cotton wick extends and the liquid is poured out on the forehead of the person. The falling liquid through a wick is causing a small, gentle impact on the forehead which generates, in combination with the defined flow volume, a suitable nerve relaxation of the forehead nerves and the parasympathetic nervous system.

After applying the liquid the same is collected in a pot below the head of the treated person and must be reheated before reusing it. Silence during the treatment is important, however traditionally practitioner will cause noises by collecting and reheating the liquid. Further, a continuous stable flow can be maintained only in rare cases with more than trained practitioner being present due to discontinuous process equipment.

Even though there have been attempts to create a device with which the treatment can be carried out without a practitioner being present, there are several disadvantages, which will be demonstrated based on US 6,402,776 B1, which shows a treatment device according to the preamble of claim 1. Firstly, the suggested devices are intended for stationary use and thus have a bulky design, which makes it difficult and time costly to transport and/or store the device. Secondly, the known prior art does not relate to the problem of noises created by the device. Thirdly, the known prior art does not teach by which technical means the lateral movement can be achieved.

### OBJECT OF THE INVENTION

It is therefore an object of the invention to provide a device for carrying out a relaxation treatment which overcomes the disadvantages of the prior art and provides an easy to use device, which is readily transportable and can be used without special knowledge of the treatment. It is a further object of the invention to provide a device which can be fully automated and which emits relatively low noises.

### DESCRIPTION OF THE INVENTION

In order to achieve the object set out above in a device for carrying out a relaxation treatment on the forehead of a person to be treated by producing a continuous liquid stream, the device comprising
- an outlet nozzle for forming the liquid stream;
- a liquid reservoir which is designed to receive the liquid stream at least partially;
- a sliding unit for reciprocating movement of the outlet nozzle in a lateral direction;

- at least one circulation pump to convey liquid from the liquid reservoir to the outlet nozzle;
- a pipe system connecting the outlet nozzle and/or the liquid reservoir with the at least one circulation pump (5);
- a housing

it is suggest according to the invention that
the housing has a base housing element and a top housing element, wherein the liquid reservoir and the at least one circulation pump are located in the base housing element; wherein the outlet nozzle and the sliding unit are located in the top housing element, wherein the pipe system has at least one flexible pipe connecting the at least one circulation pump being located in the base housing element with the outlet nozzle being located in the top housing element and that the top housing element is pivotally connected to the base housing element so that the device can be brought from a transporting state, in which the housing is collapsed, to an opened operating state, in which the top housing element is pivoted open so that a receiving volume for the head of the person to be treated is formed between the base housing element and the outlet nozzle being located in the top housing element.

The present invention relates to three different technical aspects, which create a combined synergistic solution to the objective mentioned above.

According to the first aspect all relevant units are housed in one common housing, which is divided into a base housing element and a top housing element. By arranging the liquid reservoir and the at least one circulation pump in the base housing element, which is usually the lower part of the device and is being placed on a surface during the treatment, and by arranging the sliding unit and the outlet nozzle in the top housing element, an integral design of the device is achieved, which does not require assembling before the start of the treatment.

According to the second aspect of the invention, the base housing element and the top housing element are pivotally connected, in order to allow the device to be brought from a collapsed or folded transporting state in a fully operationally operating state simply by pivoting open the top housing element, so that a receiving volume is formed between the base housing element and the top housing element in which the head of the person to be treated can be inserted. This aspect allows for a very compact and space saving design in the transporting state, in which the device can be transported or stored alike, on the one hand and enables a very easy to use concept for the user, as it can be brought into the operational operating state with one simple hand movement, on the other hand.

In order to ensure that liquid can be conveyed from the liquid reservoir being located in the base housing element to the outlet nozzle being located in the top housing element, a third aspect of the invention relates to how the pipe system is designed in order to allow pivotal relative movement of base and top housing element. Thus, the third aspect relates to the pipe system having a flexible pipe connecting the at least one circulation pump with the outlet nozzle. Due to this flexible pipe, the connection between the at least one circulation pump and the outlet nozzle is secured irrespective of the opening state in a cost efficient manner.

While there are certain advantageous embodiments of the device which have more than one circulation pumps, e.g. two, three or more separate circulation pumps, a preferred embodiment of the invention can have exactly one circulation pump, which is designed to provide the required liquid flow. Thus production cost can be reduced and the number of potential errors can be reduced.

Further in a preferred embodiment of the invention the pivotal connection between the base housing element and the top housing element is established by using at least one hinge, preferably one hinge, two hinges or at least two hinges.

In order to ensure that the volume flow of the liquid stream is sufficient to achieve the required impact force, a clear diameter of an outlet opening of the outlet nozzle can be larger than 6 mm, preferably larger than 8 mm, preferably between 8 mm and 12 mm. The cross section of the outlet opening is preferably circular.

While it is possible that each unit of the device, especially the sliding unit and/or the at least one circulation pump, has a separate control unit, which provides the specific control information to the units, if the device is switched to an ON state, it is preferred, that the device comprises a, preferably central, electronic control unit for controlling the at least one circulation pump and/or the sliding unit, so that all relevant information is processed by the electronic control unit and all relevant input signals for the units are provided by the electronic control unit. This electronic control unit can be connected to additional relevant units of the device, such as a distance sensor, a switch, a heating unit, a heating control unit, which will be described in more detail in the following. It shall not remain unsaid that while a single central electronic control unit has specific advantages with regard to the usability, it is also possible to combine a central control unit for some units with specific control units for certain units.

In a preferred embodiment of the invention the sliding unit comprises an electrical drive unit and a mechanical reciprocating unit being driven by the electrical drive unit, which mechanical reciprocating unit is designed to move the outlet nozzle in the lateral direction. By powering the sliding unit with an electrical drive unit, such as an electric motor, and having a mechanical reciprocating unit, which is designed to move the outlet nozzle, preferably in a linear motion, in the lateral direction, it is possible for the sliding unit to move the outlet nozzle in a way that the liquid stream can be applied to the forehead of the person to be treated from one side to the other and vice versa during operation.

In order to achieve a robust and cost effective design of the sliding unit while ensuring that the correct movement of the outlet nozzle is facilitated, a further preferred embodiment of the invention provides that the mechanical reciprocating unit comprises a rail with a slide to which slide the outlet nozzle is attached and a toothed belt drive being driven by the electrical drive unit to move the outlet nozzle on the rail via the slide. By the combination of the electric drive unit, preferably an electric stepper motor, and a rail-slide system, which ensures the linear reciprocation movement of the outlet nozzle, and toothed belt drive for transmission of power between the electric drive unit and the slide, precise movement of the nozzle can be achieved. In order to have a continuous motion of the outlet nozzle the rotating direction of the toothed belt drive changes periodically, in detail as soon as the maximum lateral position of the outlet nozzle is reached.

While it is in principle possible to manually adjust the length of the lateral movement of the sliding unit in order to match the width of the forehead of the person to be treated, it is preferred that the lateral movement is pre-defined in such a way that the majority of head shapes of adults is covered with only a slight excess a preferred embodiment provides that the sliding unit is designed to facilitate a lateral movement of between 100 mm and 180 mm, preferably between 110 mm and 150 mm.

In a preferred embodiment it is provided that the outlet nozzle comprises a nozzle body with an inlet opening which is connected to the pipe system, preferably the flexible pipe, and an outlet opening for forming the liquid stream during operation, wherein the outlet opening is arranged on the nozzle body so that it faces towards a treatment area of the base housing element, when the device is in the opened operating state. While it is in principle possible that the outlet nozzle is formed by an extension of the flexible pipe, it is particularly advantageous with regard to enabling the lateral movement, if the outlet nozzle has a, preferably solid, nozzle body. The nozzle body has an inlet opening, which is connected to the pipe system, and an outlet opening, wherein inlet opening and outlet opening are connected via a fluid connection. For example the nozzle body can have a bore which forms both openings and the fluid connection at the same time.

In order to ensure that the liquid stream from the outlet opening is directed to the correct position, so that it hits the forehead of the person to be treated, once the head is positioned correctly in the receiving volume, the outlet opening is arranged so that it faces a defined treatment area, where the forehead is positioned.

In a further preferred embodiment it is provided that a first angle between inlet opening and outlet opening is between 70° and 110°, preferably between 80° and 100°, more preferably 90° +/-5°. With this feature the coming liquid from the pipe system can be redirected easily, so that the liquid stream from the outlet opening is essentially perpendicular to the forehead of the person to be treated. Preferably the inlet opening is positioned on one side surface of the nozzle body whereas the outlet opening is positioned on a different side surface of the nozzle body.

In order to have a simplistic opening mechanism, which does not require additional locking mechanisms to secure the correct alignment of base housing element and top housing element, when the device is brought into the opened operating state, a preferred embodiment provides that the device comprises at least one hinge to pivotally connect the base housing element with the top housing element, wherein the at least one hinge is designed to retain the top housing element in relation to the base housing element in the opened operating state. Such a design can for example be achieved by using friction hinges or positioning hinges, which apply a torque to the top housing element that counteracts the torque exerted by the weight of the top housing element, when it is pivoted open.

It is critical to achieve the positive relaxation effect to the treated person that the distance between the outlet nozzle and the forehead person is adjusted correctly. If the distance is lower than the required distance, the impact force of the liquid to the forehead is too little while the impact force may be too high, if the distance exceeds the maximum distance. In order to ensure that the device is able to detect the actual distance between the outlet nozzle and the forehead of a person to be treated in the operating state of the device, a further preferred embodiment of the invention provides that the device further comprises a distance sensor, which distance sensor is located in the top housing element, wherein the distance sensor is configured to measure a distance between the outlet nozzle and the forehead of a person to be treated being positioned in the receiving volume. The measured distance can for example be indicated to the user by acoustic signals generated by a speaker or by optical signals such as a display.

It shall not remain unsaid that the effectively measured distance may have an implemented off-set in order to make up for different positioning of outlet nozzle and distance sensor on the top housing element.

If the pivotal movement is not performed manually but by a further motor, it is also possible that the measured distance is an input value for the further motor, in order to automatically adjust the position of the top housing element once the head of the person to be treated is placed in the receiving volume.

Even though it is advantageous to have a distance sensor that automatically measures the distance and is incorporated in the device itself, it is also possible in other embodiments that the distance can be measured by hand or any suitable measuring means or a suitable calliper. As a general rule the distance between outlet opening and the forehead of the person to be treated should be equal to the width of three to four fingers of the person to be treated.

In order to ensure the best possible measurement of the distance a further preferred embodiment of the invention provides that the distance sensor is positioned adjacent to the outlet nozzle.

To further enhance the user experience as well as to ensure safe use, especially by untrained users, it is advantageous if a feedback is given to the user with regard to the measured distance. With this information the user can adjust the position of the top housing element or the pivotal angle between top housing element and base housing element respectively until it lies within a pre-defined distance range, which is stored in the device. In order to provide this feature a preferred embodiment of the invention provides that the distance sensor is configured to generate a warning signal if the distance measured is below and/or above a predefined distance range during operation. The warning signal can for example be an audio signal generated by a speaker or an optical signal displayed on a display device. The display device may comprise a display screen and/or one or more indicators, such as e.g. coloured LEDs. It can also be advantageous to have different warning signals if the measured distance is below the predefined range and above the predefined range.

In a further preferred embodiment of the device according to the invention it is provided that the distance sensor is connected to an electronic control unit and the electronic control unit is designed to deactivate the at least one circulation pump when it receives the warning signal from the distance sensor. This specific setup enables a full automation of the device and further improves the user experience and safety as the liquid stream is interrupted or does not start, unless the measured distance is within the predefined range.

The electronic control unit referred to can either be the central electronic control unit or a specific motor control unit of the at least one circulation pump.

The design of the liquid reservoir is of great importance for the usability of the device. As different liquids may be used for carrying out the treatment it is necessary to be able to sufficiently clean the liquid reservoir after use and before storage/transportation. One possible design option is to have an exchangeable liquid tank instead of having an integrated liquid tank. This enables the user to take the liquid tank out of the device, to clean it separately and afterwards insert the cleaned liquid tank into the device. Accordingly a further preferred embodiment of the invention provides that the liquid reservoir is configured as an exchangeable liquid tank.

In order to provide better handling of the liquid tank during removal and insertion a further preferred embodiment of the invention provides that the liquid tank is connected to the pipe system via a self-closing valve in order to prevent spillage when the liquid tank is removed. The self-closing valve may contain a spring, so that the valve automatically closes once the liquid tank is removed from its connection with the pipe system.

While it is in principle possible that the head of the person rests on a separate headrest, which is not part of the device itself, it is particularly preferred that the head of the person rests directly on a surface of the device, in particular on the surface of the base housing element facing towards the top housing element. The surface can be part of the liquid reservoir or a cover element, which covers the elements of the base housing element.

In order to improve the user experience it is thus also relevant to enhance the comfort of the person to be treated. Therefore said surface may be configured as a headrest, preferably by having one or more cavities shaped to receive the back of the head or an applied padding.

Alternatively or in addition to said surface being configured as a headrest the liquid from the liquid stream has to be allowed to re-enter the liquid reservoir through said surface so that a continuous treatment can be realised by at least partially, preferably entirely, re-using the liquid during one single treatment. In other words there has to be a liquid connection between the liquid reservoir and said surface. Preferably this liquid connection is established by at least one opening, which can have any possible shape, such as e.g. circular, rectangular. Alternatively said surface can be formed by a porous textile that allows liquid to pass through, in which case no additional openings are required.

To achieve the aforesaid advantages a further preferred embodiment provides that a surface of the base housing element facing towards the top housing element is configured as a headrest and said surface has at least one opening to allow liquid coming from the outlet nozzle to re-enter the liquid reservoir.

Alternatively to having an exchangeable liquid tank, the aforementioned advantages can also be achieved if the liquid reservoir is configured as a collecting basin. A collecting basin may have a base surface and multiple wall surfaces, which enclose an open volume. In order to also satisfy the requirements of comfort and reusability of the liquid, the collecting basin is at least partially covered by an elastic fabric cover, which forms the surface of the base housing element facing towards the top housing element, which is thus also configured to be a headrest. Depending on how the elastic fabric cover is designed, it may be advantageous to design it to be exchangeable, so that it can be removed and cleaned after use, and/or to use a permeable fabric, to allow the liquid to pass through. Alternatively it is also possible to use a mesh-like fabric or to use a relatively stiff fabric cover with at least one opening.

Accordingly, a further preferred embodiment of the invention provides that the liquid reservoir is configured as a collecting basin and a, preferably exchangeable, elastic fabric element is attached to the collecting basin in order to form a surface of the base housing element facing towards the top housing element, which is configured as a headrest.

A further preferred embodiment of the invention provides that an energy source is integrated into the base housing element. The energy source may be a battery or a re-chargeable battery, which allows carrying out the treatment without being limited by availability of a connection to the power supply. Alternatively or in addition to the energy source the device may of course have an electric plug for plugging into the power grid or external battery, which may be used for operation and/or re-charging.

In order to ensure that the liquid circulating through the device during operation has the correct temperature as well as to prevent that the liquid has to be pre-heated during use, a further embodiment of the invention provides that the device further comprises a heating unit for heating the liquid and a temperature control unit for continuously measuring the temperature of the liquid, which temperature control unit is connected to the heating unit. The heating unit can for example be configured as a heating mat, which is in particular placed adjacent to the liquid reservoir or directly integrated in the liquid reservoir. The temperature control unit can for example comprise one or more temperature sensors, which for example can be integrated in the liquid reservoir and/or in the pipe system. It is also possible that heating unit and/or temperature control unit are connected to the central electronic control unit.

In addition or in alternative to the distance sensor described before, the device may also include a safety switch, which prevents the at least one circulation pump from being activated, if the device is not brought in the operating state, by using the pivotal angle between top housing element and base housing element as reference. Therefore a further embodiment of the invention provides that the at least one circulation pump is connected to a switch, which switch is configured to deactivate the at least one circulation pump in case a predefined pivotal angle between base housing element and top housing element is exceeded and/or undershot. The pivotal angle can also include an angle range, so that the switch is activated if the lower threshold of the range is undershot and if the higher threshold of the range is exceeded. The connection between the switch and the at least one circulation pump can also be established via the central electronic control unit.

As mentioned before the addition of a speaker and/or a display unit may further increase the user friendliness of the device, as important information, such as for example the distance, the operating state, the temperature of the liquid or the battery charge level, just to name a view. Therefore a further preferred embodiment of the invention provides that the device further comprises a display unit and/or a speaker.

In order to positively influence the noise level of the device during operation as well as enabling different liquids, such as water or water based liquids on the one hand and oil or oil based liquids as well as emulsions on the other hand, to be pumped while a constant liquid flow from the outlet nozzle is maintained, it is preferred to equip the device with a fitting type of circulation pumps. Intensive research and testing has shown that micropumps, which usually work on the principle of centrifugal pumps, driven by brushless-DC motors are best suited to fulfil all the above mentioned requirements. For controlling the rotational speed, said micropumps also comprise a motor control unit, which may or may not be connected to the central electronic control unit. While it is possible that the device contains two or more micropumps, in a particular preferred embodiment the devices comprises exactly one micropump.

Accordingly, a further preferred embodiment of the invention provides that the at least one circulation pump is configured as at least one micro-pump having a brushless DC motor and a motor control unit.

In a further preferred embodiment it is provided that the pipe system comprises an intake pipe, which connects the liquid reservoir with the at least one circulation pump and/or at least one filter being positioned between the liquid reservoir and the at least one circulation pump. While the intake pipe ensures that the liquid reservoir is connected with the at least one circulation pump, the filter prevents the circulation pump and the pipe system from congesting. According to a particularly preferred embodiment the filter is integrated into the intake pipe and may be designed as being exchangeable.

The invention also relates to the use of the device according to the invention for treatment of insomnia, headache, stress release, anxiety, jet lag and/or mental fatigue by subjecting the forehead of a person to a liquid stream generated by the device.

### BRIEF DESCRIPTION OF THE DRAWINGS

The invention will now be explained in more detail below with reference to exemplary embodiments. The drawings are provided by way of example and are intended to explain the concept of the invention, but shall in no way restrict it or even render it conclusively, wherein:
- Fig. 1a: shows a schematic view of a first embodiment of the device in a fully opened state;
- Fig. 1b: shows a schematic view of the device according to Fig. 1a in an opened operating state during a relaxation treatment;
- Fig. 2: shows a schematic sectional view of the device according to Fig. 1a in a collapsed transporting state;
- Fig. 3a,3b: show two embodiments of a liquid reservoir;
- Fig. 4: shows a schematic view of the interior design of a second embodiment of the device;
- Fig. 5a: shows a first sectional schematic view of a sliding unit;
- Fig. 5b: shows a schematic top view of the sliding unit;
- Fig. 5c: shows a second sectional schematic view of the sliding unit.

### WAYS OF CARRYING OUT THE INVENTION

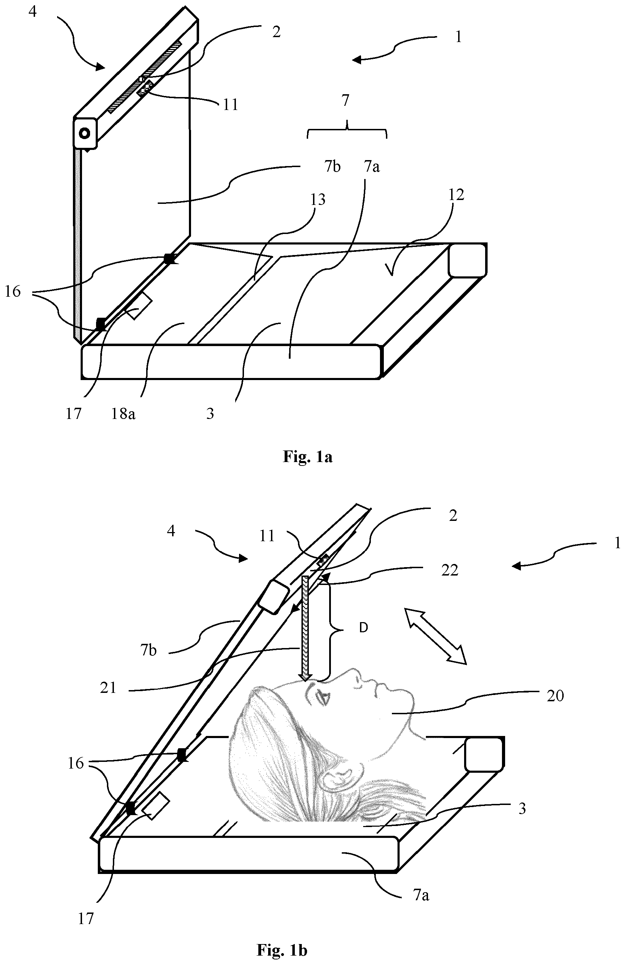

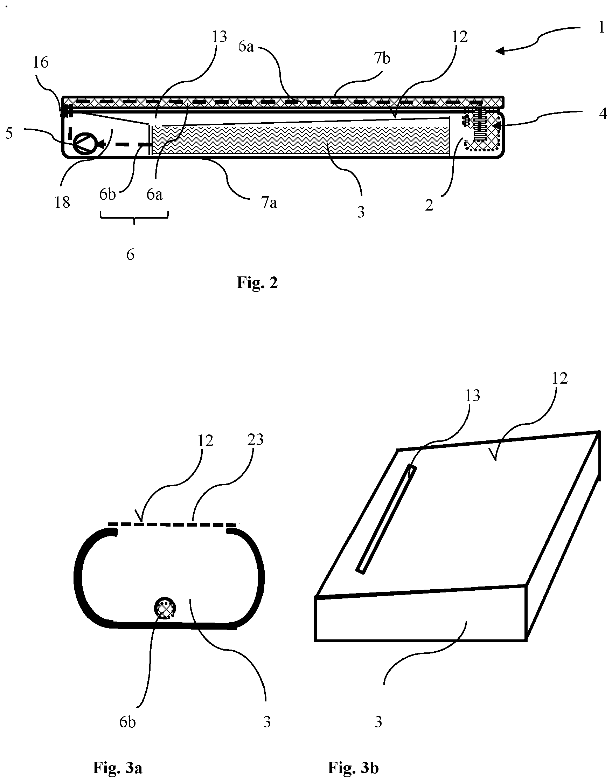

The present invention relates to a device 1 for carrying out a Shirodhara treatment which is fully functional and allows for space saving storage and easy transportation. The underlying principle of the invention shall be explained in the following based on a first exemplary embodiment of the invention shown in Figures 1a, 1b and 2.

All components required for carrying out the treatment, which are described in more detail below, are incorporated into a housing 7. The housing 7 in this particular embodiment consists of a base housing element 7a and a top housing element 7b, which are pivotally connected via two hinges 16. An outlet nozzle 2, from which a continuous liquid stream 21 emerges during the operation of the device 1 is located in the top housing element 7b. In the shown embodiment the outlet nozzle 2 is located on the end of the top housing element 7a which is opposite to the end on which the hinges 16 are attached. The length of the housing 7 is preferably less than 500 mm, especially less than 400 mm and the width of the housing 7 is preferably less than 500 mm, especially less than 450 mm.

The pivotal connection of base housing element 7a and top housing element 7b allows the device 1 to be folded open and to be collapsed in a simple and efficient way. Fig. 2 shows the device 1 in a collapsed transportation state, in which the base housing element 7a and the top housing element 7b are aligned in parallel, so that the required space is minimised. Fig. 1a shows the device 1 in a fully opened state, in which top housing element 7b and base housing element 7a are aligned essentially perpendicular to each other. Fig. 1b shows the device 1 in an opened operating state, in which base housing element 7a and top housing element 7b define a receiving volume, in which the head of a person 20 can be placed on the base housing element 7a. The head of the person 20 is further aligned so that it is located in a treatment area, so that the forehead of the person 20 faces towards the outlet nozzle 2 being located in the top housing element 7b. This design ensures that the liquid stream 21 from the outlet nozzle 2 is directed to the forehead of the person 20 to be treated. Further the distance between the forehead of the person 20 to be treated and the outlet nozzle 2 can be varied by pivotally opening or closing of the housing 7.

Figure 2 specifically shows the technical means necessary to provide a constant liquid stream 21 during treatment. The device 1 comprises a liquid reservoir 3 for storing the liquid, a circulation pump 5 which pumps the liquid from the liquid reservoir 3 to the outlet nozzle 2 and a pipe system 6, which connects liquid reservoir 3, circulation pump 5 and outlet nozzle 2.

In order to allow the housing 7 to be pivoted open or closed, the pipe system 6 comprises a flexible pipe 6a, which connects the circulation pump 5 located in the base housing element 7a and the outlet nozzle 2 being located in the top housing element 7b. Most importantly the part of the pipe system 6 which connects base housing element 7a and top housing element 7b is formed by the flexible pipe 6a. The circulation pump 5 is connected to the liquid reservoir 3 by an intake pipe 6b, which can include a filter element.

The circulation pump 5 is located in a first compartment 18 which is separated from the liquid reservoir 3 and covered by a first cover 18a.

In the present embodiment a first part surface 12 of the base housing element 7a facing towards the top housing element 7b is formed by a cover of the liquid reservoir 3, whereas a second part of said surface 12 is formed by the first cover 18a. Said surface 12 is sloped towards an opening 13, which is in fluid connection with the liquid reservoir 3, so that liquid from the liquid stream 21 can re-enter the liquid reservoir 3 so that the liquid can be constantly recycled during a relaxation treatment.

As during the classic Shirodhara treatment the liquid stream 21 is applied in a sweeping motion to the forehead of a person 20 to be treated, the device 1 further comprises a sliding unit 4, which is designed to move the outlet nozzle 2 in a lateral direction 22 from one side to the other as can be seen in Fig. 1b. The sliding unit 4 is also located in the top housing element 7b and the outlet nozzle 2 is attached to the sliding unit 4, which will be described in more detail below.

To provide a compact design the base housing element 7a can comprise a further compartment for receiving the sliding unit 4, as shown in Fig. 2, when the device 1 is in the collapsed transporting state.

Furthermore the device 1 comprises a distance sensor 11, which is located adjacent to the outlet nozzle 2. The distance sensor 11 measures the distance D between the outlet nozzle 2 and the forehead of the person 20 to be treated in order to ensure that the distance D is within a predefined range, that provides optimal effects for the treatment. Different types of distance sensors 11 can be used for example but not limited to time of flight sensors, ultrasonic sensor or IR sensors.

The distance sensor 11 can further be configured to create a warning signal and/or to deactivate the circulation pump 5, in case the measured distance D is not within the predefined range.

The device 1 further includes a display unit 17, which can e.g. indicate the measured distance D, and may additionally also include a speaker.

Figures 3a and 3b show two different embodiments of the liquid reservoir 3. In Fig. 3a the liquid reservoir 3 is integrally formed with the base housing element 7a and configured as a liquid collecting basin having multiple walls which enclose the collecting basin. The surface 12 facing towards the top housing element 7b is formed by a elastic fabric cover, preferably made of a textile, which allows liquid to pass through. This elastic fabric cover 23 is attached to the top portion of the collecting basin and can be removed and/or exchanged in order to allow easy cleaning of the liquid reservoir 3.

Fig. 3b shows a different embodiment in which the liquid reservoir 3 is configured as an exchangeable liquid tank, which can be removed from the base housing element 7a in order to allow easy cleaning and storage. The surface 12, formed by a top cover element of the liquid tank, may be specifically designed as a headrest for the person 20 to be treated. Furthermore the top cover element has at least one opening 13 to allow liquid to re-enter the liquid tank. In order to prevent spillage of liquid when the liquid tank is removed, the liquid tank can comprise a self closing valve, which is connected to the intake pipe 6.

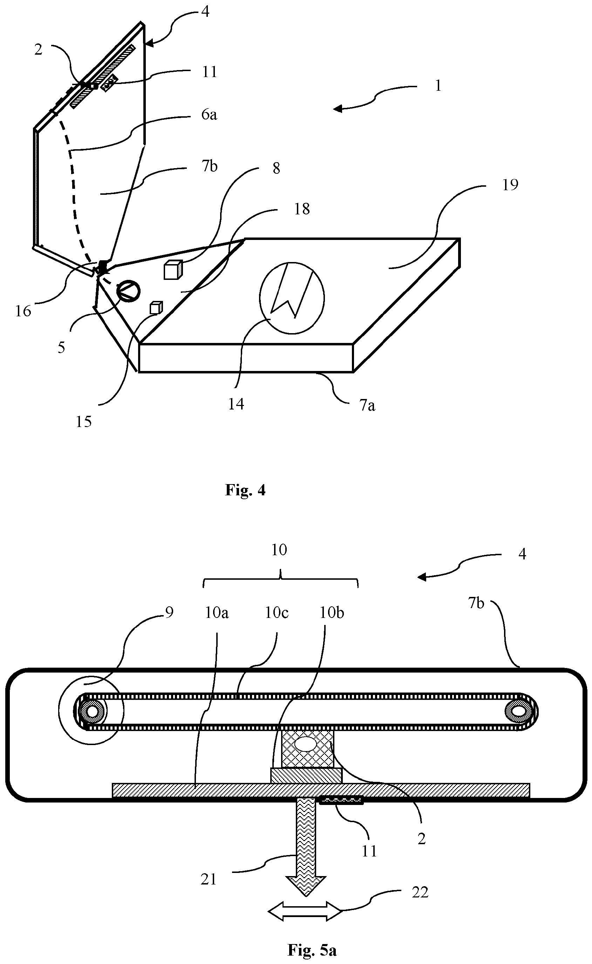

Fig. 4 schematically shows the interior design and further components of the device by way of a second exemplary embodiment. It shall be noted that the shape of the housing can be chosen relatively freely. In the shown exemplary embodiment the outline of the first compartment 18, in which the electronic components, such as e.g. the circulation pump 5, are stored, is essentially trapezoid, whereas a second compartment 19 for housing the liquid reservoir 3 is shaped rectangular. Furthermore base housing element 7a and top housing element 7b are connected via only one hinge 16.

In order to be able to heat the liquid stored in the liquid reservoir 3, the device 1 also comprises a heating unit 14, which can include one or more heating elements. The heating unit 14, for example a heating mat, is located in the second compartment 19 for housing the liquid reservoir 3 in the shown exemplary embodiment. The heating unit 14 is in the present exemplary embodiment connected to a temperature control unit 15 having one or more temperature sensors, which can directly or indirectly measure the temperature of the liquid. The temperature control unit 15 can further be designed to control the heating unit 14 so that the temperature of the liquid stays essentially constant within a predefined range, preferably around 40°C, during treatment and/or the circulation pump can only be activated once an operating temperature is reached.

The device 1 according the second exemplary embodiment has a central electronic control unit 8, which is connected to at least one, preferably all, electronic components. Preferably the electronic control unit 8 is at least controlling the circulation pump 5 and/or the sliding unit 4. Of course at least one of the temperature control unit 15 and the distance sensor 9 can also be connected to and/or controlled by the electronic control unit 8. The temperature control unit 15 can further be connected to the display unit 17 in order to indicate the current temperature or whether or not the operating temperature is reached. However it shall be noted that is also possible that multiple different components have separate control units which communicate with each other so that no central control unit is required.

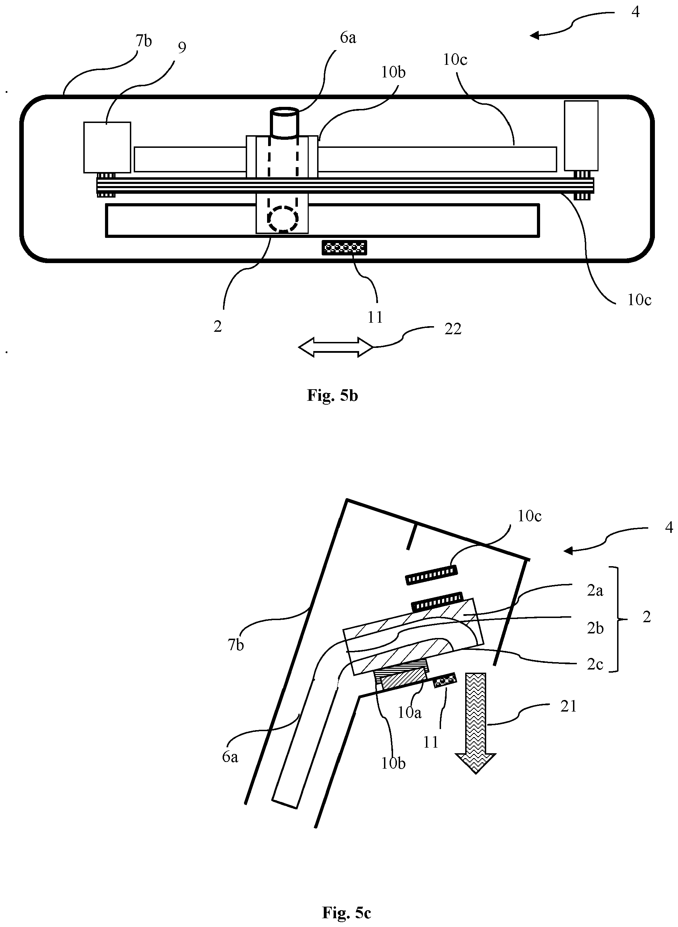

Figures 5a to 5c relate to specific constructive details of the sliding unit 4 and the outlet nozzle 2, which can be incorporated into all off the exemplary embodiments described before. In detail a top portion of the top housing element 7b is shown which is located essentially opposite to the portion of the top housing element 7b where the hinges 16 are attached.

In order to allow a smooth, silent and reliable reciprocating movement in a lateral direction 22 the sliding unit 4 comprises following elements: an electrical drive unit 9, preferably an electric motor such as a stepper motor, and a mechanical reciprocating unit 10, to which the outlet nozzle 2 is attached. The mechanical reciprocating unit 10 comprises a toothed belt drive 10c, which is powered by the electrical drive unit 9 as well as a rail 10a and a slide 10b which slides along the rail 10a. The outlet nozzle 2 is on the one hand attached to the rail 10a, which may be a metal rail, preferably made of aluminium or an aluminium alloy, via the slide 10b and on the other hand attached to the belt of the toothed belt drive 10c. The toothed belt drive 10c usually comprises the toothed belt as well as at least two gears one of which is fixed to the electrical drive unit 9.

In order to generate the movement of the outlet nozzle 2 in the lateral direction 22, the electrical drive unit 9 is controlled in such a way that the slide 10b is first moved to a first lateral end position as the electrical drive unit 9 rotates in a first direction and the toothed belt drive actuates the slide 10b. After the first lateral end position is reached the direction of rotation of the electrical drive unit 9 is changed, so that the slide 10b is moved on the rail 10a in the opposite direction until a second lateral end positon is reached. This sequence is continuously repeated, which creates a sweeping motion of the liquid stream 21 ejected from the outlet nozzle 2 to the forehead of the person 20 to be treated. Preferably the distance covered by the reciprocating movement lies between 100 mm and 180 mm, preferably between 110 mm and 150 mm.

While it is possible that the electrical drive unit 9 is controlled by the central electronic control unit 8 it is also possible that the electrical drive unit 9 has a separate control unit.

Fig. 5c shows a further detail of the present invention with regard to the design of the outlet nozzle 2 in addition to the design of the sliding unit 4 described above.

In order to ensure that the liquid stream 21 from the outlet nozzle 2 is directed to the correct position, so that it hits the forehead of the person 20 be treated, once the head is positioned correctly on the base housing element 7a, an outlet opening 2c of the outlet nozzle 2 is arranged so that it faces a defined treatment area of the base housing element 7b, where the forehead is positioned during operation. Thus the outlet nozzle 2 comprises a nozzle body 2a, which nozzle body 2a is attached to the slide 10b and engaged with the toothed belt drive 10c, with an inlet opening 2b, through which liquid can enter the outlet nozzle 2, and the outlet opening 2c, which forms the liquid stream 21. Inlet opening 2b and outlet opening 2c are connected by a fluidic connection and may be formed by a bore through the nozzle body 2a.

The inlet opening 2b is connected to the flexible pipe 6a of the pipe system 6, preferably the flexible pipe 6a is fixed to the nozzle body 2a, so that it is dragged along during the reciprocating movement of the outlet nozzle 2. To redirect the liquid stream 21 to the correct position when the device 1 is in the correct opened operating state, inlet opening 2b and outlet opening 2c are angularly offset. In the shown exemplary embodiment the angular offset is approximately 90°.

The circulation pump 5 can have a delivery volume of between 1 l/min and 3 l/min, preferably between 1,1 l/min and 2,8 l/min, more preferably between 1,2 l/min and 2,7 l/min. The circulation pump 5 can be any suitable ultra silent pump having a noise level below 20 dB (A), preferably below 15 dB (A), more preferably below 10 dB (A).

The use of centrifugal micro-pumps, which are driven by a brushless DC motor having a motor control unit, as circulation pump 5 have yielded extremely good testing results with regard to the delivery volume. In addition rubber sheets made of silicone or other polymer materials but also PU foam or other foam materials and a combination of rubber and foam can be used to additional cancel the noise of the circulation pump 5.

Even though all exemplary embodiments comprise only one circulation pump 5 it shall be mentioned that it is also possible to include two or more circulation pumps 5 in the device 1.

Furthermore with regard to all exemplary embodiments it is advantageous that the at least one hinge 16 connecting the base housing element 7a with the top housing element 7b is designed to retain the relative position of the two housing elements 7a,7b in the opened state and/or the operating state. Such a design can for example be achieved by using friction hinges or a positioning hinges, which apply a torque to the top housing element 7b in order to prevent the top housing element 7b from moving towards the base housing element 7a, when the device is pivoted open.

The device can be powered by an internal energy source (not shown), such as a battery or a rechargeable battery, or by an external energy source.

### Reference Numerals

- 1: device for carrying out a relaxation treatment
- 2: outlet nozzle
2a nozzle body
2b inlet opening
2c outlet opening
2d fluid connection

- 3: liquid reservoir
- 4: sliding unit
- 5: circulation pump
- 6: pipe system
6a flexible pipe
6b intake pipe

- 7: housing
7a base housing element
7b top housing element

- 8: control unit
- 9: electrical drive unit
- 10: mechanical reciprocating unit
10a rail
10b slide
10c toothed belt drive

- 11: distance sensor
- 12: surface of the base housing element 7a
- 13: opening
- 14: heating unit
- 15: temperature control unit
- 16: hinge
- 17: display unit
- 18: first compartment
18a first cover

- 19: second compartment
- 20: person
- 21: liquid stream
- 22: lateral direction
- 23: elastic fabric cover

- D: measured distance

## Claims

1. A device (1) for carrying out a relaxation treatment on the forehead of a person (20) to be treated by producing a continuous liquid stream (21),
the device (1) comprising
- an outlet nozzle (2) for forming the liquid stream (21);
- a liquid reservoir (3) which is designed to receive the liquid stream (21) at least partially;
- a sliding unit (4) for reciprocating movement of the outlet nozzle (2) in a lateral direction (22);
- at least one circulation pump (5) to convey liquid from the liquid reservoir (3) to the outlet nozzle (2);
- a pipe system (6) connecting the outlet nozzle (2) and/or the liquid reservoir (3) with the at least one circulation pump (5);
- a housing (7),
**characterised in that**
the housing (7) has a base housing element (7a) and a top housing element (7b),
wherein the liquid reservoir (3) and the at least one circulation pump (5) are located in the base housing element (7a);
wherein the outlet nozzle (2) and the sliding unit (4) are located in the top housing element (7b);
wherein the pipe system (6) has at least one flexible pipe (6a) connecting the at least one circulation pump (5) being located in the base housing element (7a) with the outlet nozzle (2) being located in the top housing element (7b),
**and that**
the top housing element (7b) is pivotally connected to the base housing element (7a) so that the device (1) can be brought from a transporting state, in which the housing (7) is collapsed, to an opened operating state, in which the top housing element (7b) is pivoted open so that a receiving volume for the head of the person (20) to be treated is formed between the base housing element (7a) and the outlet nozzle (2) being located in the top housing element (7b).

2. The device (1) according to claim 1, **wherein** the device (1) comprises an electronic control unit (8) for controlling the at least one circulation pump (5) and/or the sliding unit (4).

3. The device (1) according to any one of claims 1 to 2, **wherein** the sliding unit (4) comprises an electrical drive unit (9) and a mechanical reciprocating unit (10) being driven by the electrical drive unit (9), which mechanical reciprocating unit (10) is designed to move the outlet nozzle (2) in the lateral direction.

4. The device (1) according to claim 3, **wherein** the mechanical reciprocating unit (10) comprises a rail (10a) with a slide (10b) to which slide (10b) the outlet nozzle (2) is attached and a toothed belt drive (10c) being driven by the electrical drive unit (9) to move the outlet nozzle (2) on the rail (10a) via the slide (10b).

5. The device (1) according to any one of claims 1 to 4, **wherein** the outlet nozzle (2) comprises a nozzle body (2a) with an inlet opening (2b) which is connected to the pipe system (6), preferably the flexible pipe (6a), and an outlet opening (2c) for forming the liquid stream (21) during operation,
wherein the outlet opening (2c) is arranged on the nozzle body (2a) so that it faces a treatment area of the base housing element (7a), when the device (1) is in the opened operating state.

6. The device (1) according to claim 5, **wherein** a first angle between inlet opening (2b) and outlet opening (2c) is between 70° and 110°, preferably between 80° and 100°, more preferably 90° +/-5°.

7. The device (1) according to any one of claims 1 to 6,
**wherein** the device (1) comprises at least one hinge (16) to pivotally connect the base housing element (7a) with the top housing element (7b),
wherein the at least one hinge (16) is designed to retain the top housing element (7b) in relation to the base housing element (7a) in the opened operating state.

8. The device (1) according to any one of claims 1 to 7,
**wherein** the device (1) further comprises a distance sensor (11), which distance sensor (11) is located in the top housing element (7b),
wherein the distance sensor (11) is configured to measure a distance (D) between the outlet nozzle (2) and the forehead of a person (20) to be treated being positioned in the receiving volume.

9. The device (1) according to claim 8, **wherein** the distance sensor (11) is configured to generate a warning signal if the distance (D) measured is below and/or above a predefined distance range during operation
and wherein the distance sensor (11) is preferably connected to an electronic control unit, which electronic control unit is designed to deactivate the at least one circulation pump (5) when it receives the warning signal from the distance sensor (11).

10. The device (1) according to any one of claims 1 to 9,
**wherein** a surface (12) of the base housing element (7a) facing towards the top housing element (7b) is configured as a headrest and wherein said surface (12) has at least one opening (13) to allow liquid coming from the outlet nozzle (2) to re-enter the liquid reservoir (3).

11. The device (1) according to any one of claims 1 to 10, **wherein** the liquid reservoir (3) is configured as a collecting basin and a, preferably exchangeable, elastic fabric cover (23) is attached to the collecting basin in order to form a surface (12) of the base housing element (7a) facing towards the top housing element (7b), which is configured as a headrest.

12. The device (1) according to any one of claims 1 to 11, **wherein** the device (1) further comprises a heating unit (14) for heating the liquid and a temperature control unit (15) for continuously measuring the temperature of the liquid, which temperature control unit (15) is connected to the heating unit (14).

13. The device (1) according to any one of claims 1 to 12, **wherein** the at least one circulation pump (5) is connected to a switch, which switch is configured to deactivate the at least one circulation pump (5) in case a predefined pivotal angle between base housing element (7a) and top housing element (7b) is undershot.

14. The device (1) according to any one of claims 1 to 13, **wherein** the at least one circulation pump (5) is configured as at least one micro-pump having a brushless DC motor and a motor control unit.

15. The device (1) according to any one of claims 1 to 14,
**wherein** the pipe system comprises an intake pipe (6b), which connects the liquid reservoir (3) with the at least one circulation pump (5)
and/or at least one filter being positioned between the liquid reservoir (3) and the at least one circulation pump (5).

## Patentansprüche

1. Vorrichtung (1) zur Durchführung einer
Entspannungsbehandlung an der Stirn einer zu behandelnden Person (20), indem ein kontinuierlicher Flüssigkeitsstrom (21) erzeugt wird, die Vorrichtung (1) umfassend
- eine Auslassdüse (2) zum Bilden eines Flüssigkeitsstroms (21);
- einen Flüssigkeitsbehälter (3), der dafür ausgelegt ist, den Flüssigkeitsstrom (21) zumindest teilweise aufzunehmen;
- eine Gleiteinheit (4) für eine Pendelbewegung der Auslassdüse (2) in einer seitlichen Richtung (22);
- mindestens eine Umwälzpumpe (5), um Flüssigkeit aus dem Flüssigkeitsbehälter (3) zur Auslassdüse (2) zu fördern;
- ein Leitungssystem (6), das die Auslassdüse (2) und/oder den Flüssigkeitsbehälter (3) mit der mindestens einen Umwälzpumpe (5) verbindet;
- ein Gehäuse (7),
**dadurch gekennzeichnet, dass**
das Gehäuse (7) ein unteres Gehäuseelement (7a) und ein oberes Gehäuseelement (7b) aufweist,
wobei der Flüssigkeitsbehälter (3) und die mindestens eine Umwälzpumpe (5) im unteren Gehäuseelement (7a) angeordnet sind;
wobei die Auslassdüse (2) und die Gleiteinheit (4) im oberen Gehäuseelement (7b) angeordnet sind;
wobei das Leitungssystem (6) mindestens einen biegsamen Schlauch (6a) aufweist, der die mindestens eine Umwälzpumpe (5) im unteren Gehäuseelement (7a) mit der Auslassdüse (2) im oberen Gehäuseelement (7b) verbindet, **und dass**
das obere Gehäuseelement (7b) klappbar mit dem unteren Gehäuseelement (7a) so verbunden ist, dass die Vorrichtung (1) aus einem Transportzustand, in dem das Gehäuse (7) zugeklappt ist, in einen geöffneten Betriebszustand gebracht werden kann, in dem das obere Gehäuseelement (7b) aufgeklappt ist, sodass sich ein Aufnahmevolumen für den Kopf der zu behandelnden Person (20) zwischen dem unteren Gehäuseelement (7a) und der Auslassdüse (2), die im oberen Gehäuseelement (7b) angeordnet ist, bildet.

2. Vorrichtung (1) nach Anspruch 1, **wobei** die Vorrichtung (1) ein elektronisches Steuergerät (8) zum Steuern der mindestens einen Umwälzpumpe (5) und/oder der Gleiteinheit (4) umfasst.

3. Vorrichtung (1) nach einem der Ansprüche 1 bis 2, **wobei** die Gleiteinheit (4) eine elektrische Antriebseinheit (9) und eine mechanische Pendeleinheit (10), die von der elektrischen Antriebseinheit (9) angetrieben wird, umfasst, wobei die mechanische Pendeleinheit (10) dafür ausgelegt ist, die Auslassdüse (2) in der seitlichen Richtung zu bewegen.

4. Vorrichtung (1) nach Anspruch 3, **wobei** die mechanische Pendeleinheit (10) eine Schiene (10a) mit einem Schlitten (10b) umfasst, wobei an besagtem Schlitten (10b) die Auslassdüse (2) befestigt ist, und einen Zahnriemenantrieb umfasst (10c), welcher durch die elektrische Antriebseinheit (9) antreibbar ist, um die Auslassdüse (2) auf der Schiene (10a) über den Schlitten (10b) zu bewegen.

5. Vorrichtung (1) nach einem der Ansprüche 1 bis 4, **wobei** die Auslassdüse (2) einen Düsenkörper (2a) mit einer Einlassöffnung (2b) umfasst, die mit dem Leitungssystem (6) verbunden ist, bevorzugt mit dem biegsamen Schlauch (6a), und einer Auslassöffnung (2c), um im Betrieb den Flüssigkeitsstrom (21) zu bilden,
wobei die Auslassöffnung (2c) am Düsenkörper (2a) so angeordnet sind, dass sie zu einem Behandlungsbereich des unteren Gehäuseelements (7a) weist, wenn die Vorrichtung (1) im geöffneten Betriebszustand ist.

6. Vorrichtung (1) nach Anspruch 5, **wobei** ein erster Winkel zwischen Einlassöffnung (2b) und Auslassöffnung (2c) zwischen 70° und 110°, bevorzugt zwischen 80° und 100°, besonders bevorzugt zwischen 90° +/-5° liegt.

7. Vorrichtung (1) nach einem der Ansprüche 1 bis 6, **wobei** die Vorrichtung (1) mindestens ein Scharnier (16) umfasst, um das untere Gehäuseelement (7a) klappbar mit dem oberen Gehäuseelement (7b) zu verbinden,
wobei das mindestens eine Scharnier (16) dafür ausgelegt ist, das obere Gehäuseelement (7b) in Relation zum unteren Gehäuseelement (7a) im geöffneten Betriebszustand zu halten.

8. Vorrichtung (1) nach einem der Ansprüche 1 bis 7, **wobei** die Vorrichtung (1) ferner einen Abstandssensor (11) umfasst, wobei besagter Abstandssensor (11) im oberen Gehäuseelement (7b) angeordnet ist,
wobei der Abstandssensor (11) dafür konfiguriert ist, einen Abstand (D) zwischen der Auslassdüse (2) und der Stirn einer zu behandelnden Person (20) zu messen, die im Aufnahmevolumen positioniert ist.

9. Vorrichtung (1) nach Anspruch 8, **wobei** der Abstandssensor (11) dafür konfiguriert ist, ein Warnsignal zu generieren, wenn der gemessene Abstand (D) im Betrieb unter und/oder über einem vorab definierten Abstandsbereich liegt und wobei der Abstandssensor (11) bevorzugt mit einem elektronischen Steuergerät verbunden ist, wobei das elektronische Steuergerät dafür ausgelegt ist, die mindestens eine Umwälzpumpe (5) zu deaktivieren, wenn es ein Warnsignal vom Abstandssensor (11) erhält.

10. Vorrichtung (1) nach einem der Ansprüche 1 bis 9, **wobei** eine Fläche (12) des unteren Gehäuseelements (7a), die zum oberen Gehäuseelement (7b) weist, als Kopfablage konfiguriert ist und besagte Fläche (12) mindestens eine Öffnung (13) hat, die es der aus der Auslassdüse (2) austretenden Flüssigkeit ermöglicht, wieder in den Flüssigkeitsbehälter (3) zu gelangen.

11. Vorrichtung (1) nach einem der Ansprüche 1 bis 10, **wobei** der Flüssigkeitsbehälter (3) als Auffangwanne konfiguriert ist und eine, bevorzugt austauschbare, elastische Stoffabdeckung (23) an der Auffangwanne befestigt ist, um eine Fläche (12) des unteren Gehäuseelements (7a) zu bilden, die zum oberen Gehäuseelement (7b) weist und die als Kopfablage konfiguriert ist.

12. Vorrichtung (1) nach einem der Ansprüche 1 bis 11, **wobei** die Vorrichtung (1) ferner ein Heizgerät (14) zum Erwärmen der Flüssigkeit und ein Temperatursteuergerät (15) zur kontinuierlichen Messung der Temperatur der Flüssigkeit umfasst, wobei das Temperatursteuergerät (15) mit dem Heizgerät (14) verbunden ist.

13. Vorrichtung (1) nach einem der Ansprüche 1 bis 12, **wobei** die mindestens eine Umwälzpumpe (5) mit einem Schalter verbunden ist, wobei der Schalter dafür konfiguriert ist, die mindestens eine Umwälzpumpe (5) im Fall, dass ein vorab definierter Aufklappwinkel zwischen dem oberen Gehäuseelement (7a) und dem unteren Gehäuseelement (7b) unterschritten wird, zu deaktivieren.

14. Vorrichtung (1) nach einem der Ansprüche 1 bis 13, **wobei** die mindestens eine Umwälzpumpe (5) als mindestens eine Mikropumpe mit einem bürstenlosen Gleichstrommotor und einem Motorsteuergerät konfiguriert ist.

15. Vorrichtung (1) nach einem der Ansprüche 1 bis 14, **wobei** das Leitungssystem ein Einlassrohr (6b), das den Flüssigkeitsbehälter (3) mit der mindestens einen Umwälzpumpe (5)
und/oder mindestens einem Filter, der zwischen dem Flüssigkeitsbehälter (3) und der mindestens einen Umwälzpumpe (5) positioniert ist, verbindet.

## Revendications

1. Dispositif (1) pour réaliser un traitement de relaxation sur le front d'une personne (20) à traiter en produisant un flux de liquide continu (21),
le dispositif (1) comprenant
- une buse de sortie (2) pour former le flux de liquide (21) ;
- un réservoir de liquide (3) qui est conçu pour recevoir le flux de liquide (21) au moins partiellement ;
- une unité coulissante (4) pour le mouvement de va-et-vient de la buse de sortie (2) dans une direction latérale (22) ;
- au moins une pompe de circulation (5) pour convoyer le liquide depuis le réservoir de liquide (3) jusqu'à la buse de sortie (2) ;
- un système de tuyauterie (6) reliant la buse de sortie (2) et/ou le réservoir de liquide (3) à l'au moins une pompe de circulation (5) ;
- un boîtier (7),
**caractérisé en ce que**
le boîtier (7) a un élément de boîtier de base (7a) et un élément de boîtier de dessus (7b), dans lequel le réservoir de liquide (3) et l'au moins une pompe de circulation (5) sont situés dans l'élément de boîtier de base (7a) ;
dans lequel la buse de sortie (2) et l'unité coulissante (4) sont situées dans l'élément de boîtier de dessus (7b) ;
dans lequel le système de tuyauterie (6) a au moins un tuyau flexible (6a) reliant l'au moins une pompe de circulation (5) située dans l'élément de boîtier de base (7a) avec la buse de sortie (2) située dans l'élément de boîtier de dessus (7b),
**et en ce que**
l'élément de boîtier de dessus (7b) est relié de manière pivotante à l'élément de boîtier de base (7a) de telle sorte que le dispositif (1) peut être amené depuis un état de transport, dans lequel le boîtier (7) est replié, dans un état de fonctionnement ouvert, dans lequel l'élément de boîtier de dessus (7b) pivote pour s'ouvrir de telle sorte qu'un volume de réception pour la tête de la personne (20) à traiter est formé entre l'élément de boîtier de base (7a) et la buse de sortie (2) située dans l'élément de boîtier de dessus (7b).

2. Dispositif (1) selon la revendication 1, **dans lequel** le dispositif (1) comprend une unité de commande électronique (8) pour commander l'au moins une pompe de circulation (5) et/ou l'unité coulissante (4).

3. Dispositif (1) selon l'une quelconque des revendications 1 à 2, **dans lequel** l'unité coulissante (4) comprend une unité d'entraînement électrique (9) et une unité de va-et-vient mécanique (10) entraînée par l'unité d'entraînement électrique (9), laquelle unité de va-et-vient mécanique (10) est conçue pour déplacer la buse de sortie (2) dans la direction latérale.

4. Dispositif (1) selon la revendication 3, **dans lequel** l'unité de va-et-vient mécanique (10) comprend un rail (10a) avec une coulisse (10b) à laquelle coulisse (10b) la buse de sortie (2) est fixée et un entraînement à courroie crantée (10c) entraînée par l'unité d'entraînement électrique (9) pour déplacer la buse de sortie (2) sur le rail (10a) par le biais de la coulisse (10b).

5. Dispositif (1) selon l'une quelconque des revendications 1 à 4, **dans lequel** la buse de sortie (2) comprend un corps de buse (2a) avec une ouverture d'entrée (2b) qui est reliée au système de tuyauterie (6), préférentiellement le tuyau flexible (6a), et une ouverture de sortie (2c) pour former le flux de liquide (21) pendant le fonctionnement,
dans lequel l'ouverture de sortie (2c) est agencée sur le corps de buse (2a) de telle sorte qu'elle est orientée face à une zone de traitement de l'élément de boîtier de base (7a), dans lequel le dispositif (1) est dans l'état de fonctionnement ouvert.

6. Dispositif (1) selon la revendication 5, **dans lequel** un premier angle entre l'ouverture d'entrée (2b) et l'ouverture de sortie (2c) est compris entre 70° et 110°, préférentiellement entre 80° et 100°, plus préférentiellement de 90° +/-5°.

7. Dispositif (1) selon l'une quelconque des revendications 1 à 6, **dans lequel** le dispositif (1) comprend au moins une charnière (16) pour relier de manière pivotante l'élément de boîtier de base (7a) avec l'élément de boîtier de dessus (7b),
dans lequel l'au moins une charnière (16) est conçue pour retenir l'élément de boîtier de dessus (7b) en lien avec l'élément de boîtier de base (7a) dans l'état de fonctionnement ouvert.

8. Dispositif (1) selon l'une quelconque des revendications 1 à 7, **dans lequel** le dispositif (1) comprend en outre un capteur de distance (11), lequel capteur de distance (11) est situé dans l'élément de boîtier de dessus (7b),
dans lequel le capteur de distance (11) est conçu pour mesurer une distance (D) entre la buse de sortie (2) et le front d'une personne (20) à traiter positionné dans le volume de réception.

9. Dispositif (1) selon la revendication 8, **dans lequel** le capteur de distance (11) est conçu pour générer un signal d'avertissement si la distance (D) mesurée est inférieure et/ou supérieure à une plage de distance prédéfinie pendant le fonctionnement
et dans lequel le capteur de distance (11) est préférentiellement relié à une unité de commande électronique, laquelle unité de commande électronique est conçue pour désactiver l'au moins une pompe de circulation (5) lorsqu'elle reçoit le signal d'avertissement depuis le capteur de distance (11).

10. Dispositif (1) selon l'une quelconque des revendications 1 à 9, **dans lequel** une surface (12) de l'élément de boîtier de base (7a) faisant face à l'élément de boîtier de dessus (7b) est conçue comme appui-tête et dans lequel ladite surface (12) a au moins une ouverture (13) pour permettre à du liquide sortant de la buse de sortie (2) de réentrer dans le réservoir de liquide (3).

11. Dispositif (1) selon l'une quelconque des revendications 1 à 10, **dans lequel** le réservoir de liquide (3) est conçu en tant que bassine de collecte et un couvercle en tissu élastique (23), préférentiellement échangeable, est fixé à la bassine de collecte afin de former une surface (12) de l'élément de boîtier de base (7a) faisant face à l'élément de boîtier de dessus (7b), qui est conçu comme appui-tête.

12. Dispositif (1) selon l'une quelconque des revendications 1 à 11, **dans lequel** le dispositif (1) comprend en outre une unité de chauffage (14) pour chauffer le liquide et une unité de commande de température (15) pour mesurer en continu la température du liquide, laquelle unité de commande de température (15) est reliée à l'unité de chauffage (14).

13. Dispositif (1) selon l'une quelconque des revendications 1 à 12, **dans lequel** l'au moins une pompe de circulation (5) est reliée à un commutateur, lequel commutateur est conçu pour désactiver l'au moins une pompe de circulation (5) dans le cas où un angle de pivotement prédéfini entre l'élément de boîtier de base (7a) et l'élément de boîtier de dessus (7b) n'est pas atteint.

14. Dispositif (1) selon l'une quelconque des revendications 1 à 13, **dans lequel** l'au moins une pompe de circulation (5) est conçue en tant qu'au moins une micro-pompe ayant un moteur CC sans balais et une unité de commande de moteur.

15. Dispositif (1) selon l'une quelconque des revendications 1 à 14, **dans lequel** le système de tuyauterie comprend un tuyau d'entrée (6b), qui relie le réservoir de liquide (3) à l'au moins une pompe de circulation (5)
et/ou au moins un filtre positionné entre le réservoir de liquide (3) et l'au moins une pompe de circulation (5).
